# FASCICULE DE BREVET EUROPEEN

(11) **EP 3 924 305 B1**
(45) Date de publication et mention de la délivrance du brevet: **25.09.2024**
(21) Numéro de dépôt: 20704297.9
(22) Date de dépôt: 12.02.2020
(51) Int. Cl.: C02F 3/10, C02F 3/28, C12M 1/12

(54) **SUPPORT DE CULTURE POUR PROCÉDÉ MÉTHANISATION**
KULTURSUBSTRAT FÜR METHANISIERUNGSVERFAHREN
CULTURE SUBSTRATE FOR METHANISATION METHOD

(30) Priorité: 15.02.2019 FR 1901540
(43) Date de publication de la demande: 22.12.2021
(73) Titulaire: Biorengaz, 67100 Strasbourg (FR)
(72) Inventeur: FRITSCH, Jonathan, 67100 STRASBOURG (FR)
(74) Mandataire: Casalonga
(86) Numéro de dépôt international: PCT/EP2020/053632
(87) Numéro de publication internationale: WO 2020/165271

(56) Documents cités:
- CN-B- 1 772 653
- JP-A- 2002 219 442
- NONE: "Empilement de carrés dans un cercle", 1 June 2023 (2023-06-01), XP093050987, Retrieved from the Internet <URL:https://fr.wikipedia.org/wiki/Empilement_de_carr%C3%A9s_dans_un_cercle> [retrieved on 20230601]
- JOUBERT NELLY: "Compost de Déchets Verts", 30 September 2012 (2012-09-30), pages 1 - 4, XP093132855, Retrieved from the Internet <URL:chrome-extension://efaidnbmnnnibpcajpcglclefindmkaj/https://paca.chambres-agriculture.fr/fileadmin/user_upload/National/FAL_commun/publications/Provence-Alpes-Cote_d_Azur/agriculture_biologique/compost_des_dechets_verts.pdf> [retrieved on 20240219]
- RALPH W ROBINSON ET AL: "Light and Electron Microscopic Examinations of Methane-Producing Biofilms from Anaerobic Fixed-Bed Reactorst", APPLIED AND ENVIRONMENTAL MICROBIOLOGY, 31 July 1984 (1984-07-31), pages 127 - 136, XP055627400, Retrieved from the Internet <URL:https://aem.asm.org/content/aem/48/1/127.full.pdf> [retrieved on 20190930]

## Description

La présente invention se situe dans le domaine de la valorisation des déchets. Elle concerne plus particulièrement un procédé de préparation d'une unité de méthanisation.

La méthanisation est le processus naturel biologique de dégradation de la matière organique en absence d'oxygène. Cette dégradation produit un biogaz, utilisable comme source d'énergie. La méthanisation est aussi appelée digestion anaérobie, qui peut comprendre une étape de dégradation de matières biologique en CH₄ et CO₂ et/ou une étape de transformation de CO₂ et H₂ en CH₄.

La méthanisation liquide classique infiniment mélangée (ou CSTR, « Continuous Stirred Tank Reactor ») est actuellement la méthode de méthanisation la plus répandue. Celle-ci a l'avantage d'avoir un grand retour d'expérience et la capacité d'incorporer une grande variabilité d'intrant, mais ne présente que de faibles performances par unité de volume.

Les méthanisations liquides à lit de boue granulaire (EGSB, « Expanded Granular Sludge Bed » ou UASB, « Up-flow Anaérobie Sludge Blanket ») mettent en oeuvre des réacteurs dans lesquels les boues ou les granules de bactéries sont mises en suspension par une recirculation du milieu. Ce sont des technologies compactes à hautes performances permettant la rétention de biomasse et traitant des effluents liquides sans matière en suspension. Les performances de ces procédés seraient impactées négativement par la présence de matière en suspension (MES) et de corps gras présents dans la pulpe de biodéchets.

Les méthanisations liquides équipées de supports de culture fixes ou mobiles présentent de hauts rendements de traitement pour les effluents liquides et assurent une résistance accrue aux inhibiteurs mais sont également soumises à différentes contraintes réduisant leurs performances. Les supports de culture fixes sont fréquemment obstrués ou bouchés par des dépôts biologiques et/ou minéraux nécessitant des opérations de nettoyage ou de remplacement à l'intérieur des digesteurs. Les supports de culture mobiles connaissent également ce phénomène réduisant leur performance par diminution de la surface spécifique et sont également soumis à une abrasion les détériorant et induisant une durée de vie limitée. Dans les deux cas les opérations de nettoyage et/ou de remplacement induisent des pertes de productions considérables de même que des opérations de maintenance fastidieuses et cela même avant d'aborder le coût très important de ces supports de culture lorsqu'il s'agit d'équiper de grands volumes. Il est courant que les supports de culture utilisés pour la méthanisation soient réalisés en matière plastique. De telles solutions sont chères, issues de ressources fossiles, et génèrent des déchets plastiques nocifs pour l'environnement.

La méthanisation par voie sèche continue pourrait permettre d'intégrer le support de culture simultanément aux biodéchets, mais en pratique, les retours d'expériences mettent en évidence une consommation énergétique importante et de nombreux problèmes de casse machine (incorporateurs, agitateurs, pompes, ...) bloquant le fonctionnement du digesteur. L'accumulation de sédiments (pierres, sable, indésirables) à l'intérieur des digesteurs réduit les performances et nécessite des opérations de maintenance curative fastidieuses nécessitant l'arrêt du digesteur affecté et engendrant des pertes d'exploitation conséquentes.

La méthanisation par voie sèche discontinue, dans laquelle la cuve est alimentée au chargeur en matières solides brutes dans des tunnels/garages, est une autre méthode existante. L'absence de système d'agitation et/ou d'incorporation dans les tunnels évite les contraintes mécaniques précitées (casse, usure prématurée). Toutefois l'absence de prétraitement mécanique (broyage) des matières traitées ralentit considérablement la cinétique de dégradation, minimisant les performances. Les problèmes de rhéologie liés à une porosité insuffisante et/ou non homogène de la matière incorporée perturbent fréquemment les équilibres réactionnels et le fonctionnement optimal des installations.

Il est courant que les supports de culture utilisés pour la méthanisation soient réalisés en matière plastique. De telles solutions sont chères, et génèrent des déchets plastiques nocifs pour l'environnement.

Le document EP1818314 divulgue un procédé et installation de production de biogaz à partir de liquides chargés en matière organique, utilisant un support de culture en copeaux de bois., Or l'entrainement d'un tel support par la matière en suspension du liquide traité provoque son agglomération, puis son occlusion et ne permet pas d'obtenir une méthanisation efficace dans la durée.

L'article « Light and électron microscopie examinations of methane-producing biofilms from anaérobie fixed-bed reactors » de Robinson R. W. et al (Appl. Environ. Microbiol., 1984, 48(1), pp 127-136) concerne une étude sur les biofilms producteurs de méthane. Cet article divulgue un exemple d'utilisation de morceaux de pin comme support de culture dans un digesteur mis en oeuvre dans un procédé de méthanisation d'effluents liquides.

La demande JP 2002 219442 concerne des supports de culture naturels pour un procédé de méthanisation.

La demande CN 1772653 porte sur un garnissage de réacteur anaérobique à base de bambou pour le traitement des eaux usées.

Un objet de la présente invention est de proposer un procédé de préparation d'une unité de méthanisation permettant une méthanisation rapide, efficace et pérenne d'effluents liquides, qui soit peu cher et respectueux de l'environnement.

La présente invention a pour objet un procédé de préparation d'une unité de méthanisation, comportant dans l'ordre les étapes suivantes :
- la collecte de déchets verts,
- le compostage desdits déchets vert,
- le criblage des déchets verts issu du compostage, le criblage se faisant à une dimension comprise entre 30 et 80mm de sorte à éliminer les particules fines de déchets verts,
- l'insertion des déchets verts issus du criblage dans ladite unité de méthanisation, de sorte à former un garnissage structuré, qui est un support de culture 1 constitué à plus de 50 % d'éléments bois 2 dont au moins une dimension est supérieure à 80 mm, la porosité dudit support de culture 1 étant supérieure à 50%.

Grâce à ces dispositions, un support de culture 1 efficace et pérenne pour la méthanisation peut être obtenu de façon simple et peu chère selon le procédé de l'invention ci-dessus, avec un matériau respectueux de l'environnement.

Selon d'autres caractéristiques :
- lesdits éléments bois 2 peuvent comporter des branches ramifiées et/ou gauches, ces éléments étant faciles à trouver et permettant de valoriser les déchets verts,
- le procédé de préparation peut comporter une étape de broyage après l'étape de collecte et avant l'étape d'insertion, le broyage étant un broyage lent, de sorte à obtenir qu'une majorité d'éléments broyés ait une plus grande dimension inférieure au mètre, ce qui permet de ne plus avoir d'éléments trop grands pour intégrer le support de culture,

- le procédé de préparation comporte une étape de criblage avant l'étape d'insertion, le criblage se faisant à une dimension comprise entre 30 et 80 mm, ce qui permet d'éliminer les particules fines de déchets verts qui ne seraient pas stables par méthanisation, qui réduiraient la porosité du support de culture et qui risqueraient de le boucher,
- le procédé de préparation comporte une étape de compostage avant l'étape de criblage, ce qui permet lors du criblage de séparer du compost les déchets verts non dégradables par méthanisation, notamment le bois, qui formeront un support de culture stable dans le temps.

La présente invention concerne également un procédé de méthanisation d'effluents liquides à garnissage structuré, ledit procédé comprenant :
- la préparation d'une unité de méthanisation par un procédé selon l'invention, puis
- une étape de méthanisation d'effluents liquides dans ladite unité de méthanisation.

La présente invention concerne également un procédé de méthanisation comportant dans l'ordre les étapes suivantes :
- insertion d'un support de culture selon l'invention dans un tunnel de méthanisation amont,
- envoi d'effluents liquides dans le tunnel de méthanisation amont,
- insertion d'un support de culture selon l'invention dans un tunnel de méthanisation aval,
- envoi des effluents traités en sortie du tunnel de méthanisation amont dans le tunnel de méthanisation aval.

Le fonctionnement séquentiel décalé dans le temps du procédé permet une méthanisation en continu des effluents liquides, même lorsqu'un des tunnels est arrêté. De plus l'inoculation du nouveau support par l'effluent traité sur le support en fonctionnement permet une colonisation très rapide des supports de culture, en plaçant le tunnel ayant un nouveau support de culture en aval d'un tunnel déjà en fonctionnement.

Selon d'autres caractéristiques :
- une partie comprise entre 30 et 60% des effluents traités en sortie du tunnel de méthanisation aval peut être renvoyée en entrée du tunnel de méthanisation amont, ce qui permet une dilution des effluents avant leur méthanisation et/ou une inoculation du nouveau support, et ainsi une méthanisation plus rapide.

La présente invention concerne également l'utilisation pour un procédé de méthanisation d'effluents liquides à garnissage structuré d'un support de culture 1 obtenu par le procédé suivant :
- la collecte de déchets verts,
- le compostage desdits déchets vert,
- le criblage des déchets verts issu du compostage, le criblage se faisant à une dimension comprise entre 30 et 80mm de sorte à éliminer les particules fines de déchets verts,
- formation dudit support de culture 1 à partir des déchets verts issus du criblage,

ledit support de culture 1 étant constitué à plus de 50% d'éléments bois 2, dont au moins une dimension est supérieure à 80 mm, la porosité dudit support de culture 1 étant supérieure à 50%, et
ledit procédé de méthanisation se faisant dans une unité de méthanisation et comprenant une étape de préparation de ladite unité de méthanisation dans laquelle le support de culture 1 est inséré dans ladite unité de méthanisation de sorte à former un garnissage structuré.

La présente invention sera mieux comprise à la lecture de la description détaillée qui fait suite, en référence aux figures annexées dans lesquelles :
La figure 1 est une vue schématique d'un élément bois 2 d'un support de culture 1 selon un mode de réalisation préféré de l'invention.
La figure 2 est une vue schématique d'un élément bois 2 d'un support de culture 1 selon un deuxième mode de réalisation de l'invention.
La figure 3 est une vue schématique d'un élément bois 2 d'un support de culture 1 selon un troisième mode de réalisation de l'invention.
La figure 4 est une vue schématique d'une installation de méthanisation selon un mode de réalisation préféré de l'invention.

Le support de culture 1 selon l'invention est destiné à être utilisé dans un procédé de méthanisation d'effluents liquides à garnissage structuré. Le support de culture 1 est constitué à plus de 50 % d'éléments bois 2 dont au moins une dimension est supérieure à 80 mm.

La dimension des éléments bois 2 permet au support de culture 1 d'avoir une porosité élevée, ce qui favorise l'écoulement des fluides et l'obtention d'une surface d'échange élevée pour le développement bactérien d'une part, et entre le support de culture et les effluents liquides à traiter d'autre part. On améliore encore cette porosité en utilisant des éléments bois de formes diverses et non régulières. Si les éléments sont de plus petites dimensions, ils s'imbriquent plus facilement en laissant peu d'espace entre eux, ce qui résulte dans la plupart des cas en un support de culture 1 ne permettant pas un écoulement suffisant des fluides. Ceci peut provoquer une compaction du support, voire une occlusion, diminuant la surface d'échange.

Dans la présente invention, le terme « porosité », appliqué à un support de culture, s'entend comme la fraction de volume occupée par de l'air dans le volume total du support de culture.

La mesure de la porosité peut par exemple être effectuée de la façon suivante :
- on dispose le support de culture 1 dans un contenant,
- on remplit le contenant d'eau, suffisamment rapidement pour que l'eau n'ait pas le temps de s'infiltrer à l'intérieur des éléments composant le support de culture 1, notamment les éléments bois 2, afin que la porosité interne à ces éléments ne soit pas prise en compte,
- la porosité est alors égale au volume d'eau ajoutée dans le contenant, divisé par le volume du contenant.

La porosité du support de culture 1 est de préférence supérieure à 50%, ce qui permet une surface d'échange satisfaisante pour l'efficacité du procédé de méthanisation entre d'une part le support de culture 1 et les bactéries qu'il abrite, et d'autre part les effluents liquides. Une telle porosité permet également de réduire les risques que le support de culture 1 ne soit obstrué ou bouché par des dépôts biologiques et/ou minéraux, nécessitant des opérations de nettoyage ou remplacement du support de culture 1. On obtient ainsi une plus grande durée de vie du support de culture 1.

Une plus grande porosité permet, jusqu'à un certain point, le maintien d'un écoulement suffisant avec le maintien de la surface d'échange, ou surface disponible pour le développement de bactéries, ce qui permet une plus grande efficacité de la réaction de méthanisation. C'est bien le maintien de la surface d'échange qui permet la plus grande efficacité, mais elle est très difficile à mesurer ; c'est pourquoi la porosité est utilisée pour caractériser un support de culture efficace.

Des exemples de porosités mesurées donnent des valeurs de 63, 65, et 70 %. Ce sont effectivement des valeurs en moyenne plus élevées que pour des copeaux de bois, où la porosité est généralement comprise entre 40 et 60 %.

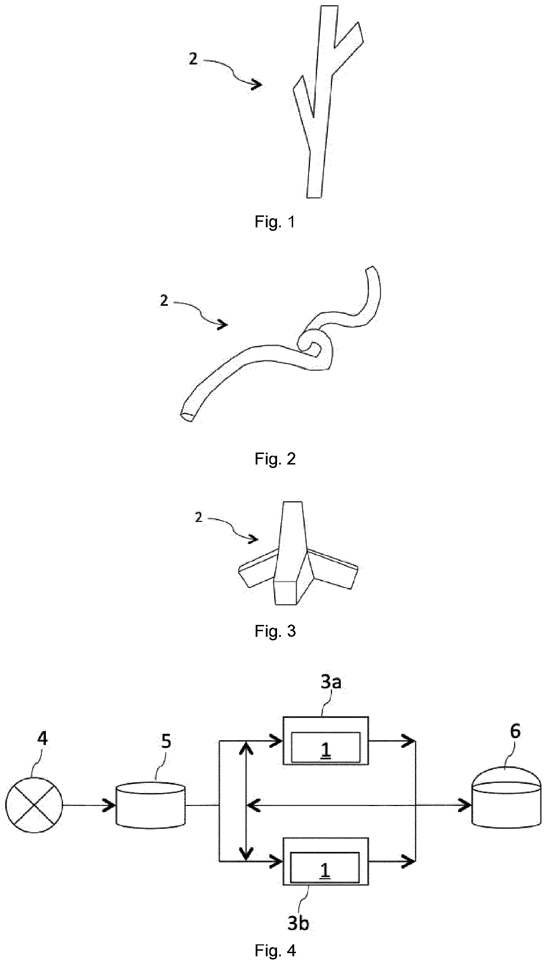

Dans un mode de réalisation préféré de l'invention, les éléments bois 2 sont des branches ramifiées et/ou gauches. Dans le cadre de la présente invention, l'expression « branche ramifiée » désigne une branche comportant au moins une ramification, c'est-à-dire qu'elle comporte au moins deux parties linéaires, pas forcément droites, formant un angle entre elles. Un exemple est illustré en fig. 1. L'expression « branche gauche » désigne une branche dont la forme est telle qu'elle n'est pas contenue dans un plan. Un exemple est illustré en fig. 2.

Les éléments bois 2 de type branches ramifiées et/ou gauche présentent l'avantage quand on les superpose, de laisser des espaces libres entre eux, de sorte qu'on obtient une porosité globale du support de culture 1 plus élevée et une surface d'échange plus élevée.

Ils sont par ailleurs faciles à trouver et peu chers : il peut s'agir par exemple de déchets verts constitués en majorité de bois, ou encore de la partie non dégradée d'un compostage de déchets verts, qu'on peut récupérer en sortie du compostage. Il peut s'agir par exemple du refus de criblage du compost.

Dans la présente invention, le terme « déchets verts » désigne l'ensemble des matières issues de végétaux ligno-cellulosiques et provenant de la taille, de la coupe ou de l'entretien de ces végétaux, par exemple lors de l'entretien de jardins, d'espaces verts, de forêts, de haies, ou encore d'arbres. Les déchets verts peuvent aussi inclure les sous-produits issus de la transformation et de la valorisation du bois, un sous-produit étant une substance ou un objet issu d'un processus de production dont le but premier n'est pas la production de cette substance ou de cet objet.

D'autres types d'éléments bois 2 peuvent être utilisés, tant que leur forme assure une porosité importante au support de culture et une bonne surface d'échange. Il peut s'agir par exemple d'éléments en forme de tétrapode, comme illustré en fig. 3, de plaquettes forestières, de broyats, ou d'une autre forme encore.

Le support de culture 1 préparé au cours du procédé de préparation d'une unité de méthanisation selon l'invention est obtenu de façon peu chère, localement, et permet une valorisation des déchets verts.

Une étape de broyage peut être mise en oeuvre entre l'étape de collecte et l'étape d'insertion. Le broyage est alors un broyage lent, de sorte à obtenir qu'une majorité d'éléments broyés ait une plus grande dimension inférieure au mètre. Ce broyage peut être nécessaire si les déchets verts collectés sont de trop grandes dimensions, ce qui peut poser problème pour l'étape d'insertion, ou ce qui peut résulter en un support de culture 1 trop poreux, la présence de grosses branches empêchant l'introduction de branches plus fines. Un support de culture 1 trop poreux, par exemple supérieure à 90%, résulte en une surface d'échange réduite entre les effluents à traiter et les bactéries, et dégrade donc les performances du procédé de méthanisation.

Selon l'invention, une étape de criblage est mise en oeuvre avant l'étape d'insertion, après le broyage s'il y en a un. Le criblage, qui se fait à une dimension comprise entre 30 et 80 mm, permet de se débarrasser des particules fines qui participeraient à l'occlusion du support de culture, ce qui réduirait l'efficacité du procédé de méthanisation.

Selon l'invention, une étape de compostage a lieu avant l'étape de criblage. Ceci permet de dégrader les déchets verts pour qu'une fois le compost éliminé par l'étape de criblage, il ne reste que les matières non dégradables par compost, riches en lignine. Ces matières riches en lignine étant non, ou très peu, dégradables par méthanisation, elles permettront d'obtenir un support de culture 1 stable.

Le support de culture 1 est ainsi peu cher, obtenu localement et respectueux de l'environnement, et permet d'obtenir de bonnes performances lorsqu'il est utilisé dans un procédé de méthanisation.

Le support de culture 1 peut être utilisé dans un procédé de méthanisation en continu, illustré en fig. 4 et comportant dans l'ordre les étapes suivantes :
- insertion d'un support de culture 1 dans un tunnel de méthanisation amont 3a,
- envoi d'effluents liquides dans le tunnel de méthanisation amont 3a,
- insertion d'un support de culture 1 dans un tunnel de méthanisation aval 3b,
- envoi des effluents traités en sortie du tunnel de méthanisation amont 3a en entrée du tunnel de méthanisation aval 3b.

Depuis sa mise en fonction, le support de culture 1 du tunnel de méthanisation amont 3a a eu le temps d'être colonisé par les bactéries. Les effluents envoyés dans le tunnel de méthanisation aval 3b sont alors chargés en bactéries, et la colonisation du tunnel de méthanisation aval 3b est très rapide, ce qui améliore les performances du procédé de méthanisation.

Lorsque le tunnel de méthanisation amont 3a est en fin de vie, le tunnel de méthanisation aval 3b, dont le support de culture 1 est plus jeune, continue à fonctionner tout seul, le temps de remplacer le support de culture 1 dans le premier tunnel de méthanisation 3a. Le tunnel de méthanisation aval 3b devient alors le tunnel de méthanisation amont 3a, et vice versa. Le procédé se répète ainsi en continu, chaque tunnel de méthanisation 3a, 3b ayant tour à tour un support de culture 1 plus vieux que l'autre tunnel de méthanisation 3b, 3a, le tunnel de méthanisation 3a, 3b ayant le support de culture le plus vieux étant placé en amont de l'autre tunnel de méthanisation 3b, 3a.

L'usage de deux tunnels de méthanisation 3a, 3b permet d'avoir un procédé fonctionnant sans interruption, pas seulement lors de la fin de vie d'un des tunnels de méthanisation 3a, 3b, mais également lors d'opérations de maintenance ou lorsqu'on évacue des indésirables d'un des tunnels de méthanisation 3a, 3b.

Dans un mode de réalisation préféré de l'invention, une partie comprise entre 30 et 60% des effluents traités en sortie du tunnel de méthanisation aval 3b sont renvoyés dans le tunnel de méthanisation amont 3a. Ceci permet une dilution des effluents en entrée, qui sont parfois trop épais pour permettre une méthanisation rapide et efficace.

Une telle dilution peut également être mise en oeuvre lorsque le tunnel de méthanisation amont 3a fonctionne seul, notamment avant la mise en oeuvre du tunnel de méthanisation aval 3b. On a alors une partie comprise entre 30 et 60% des effluents traités en sortie du tunnel de méthanisation amont 3a renvoyés en entrée du tunnel de méthanisation amont 3a.

Comme illustré en fig. 4, avant la méthanisation dans les tunnels de méthanisation 3a, 3b le procédé de méthanisation selon l'invention peut comporter une des deux étapes suivantes :
- préparation dans un déconditionneur et/ou broyeur 4,
- hygiénisation dans un hygiénisateur 5.

Après méthanisation, le biogaz peut faire l'objet d'un post-stockage dans un gazomètre 6.

Le procédé de méthanisation selon l'invention permet que le temps de séjour des solides (TSS) dans les tunnels de méthanisation 3a, 3b soit maximisé. On obtient ainsi une colonisation optimale du support et la conservation des bactéries d'intérêt par découplage du temps de séjour des effluents liquides (biodéchets) de celui des solides (bactéries / support de culture).

Bien que la description ci-dessus se base sur des modes de réalisation particuliers, elle n'est nullement limitative de la portée de l'invention, et des modifications peuvent être apportées, notamment par substitution d'équivalents techniques ou par combinaison différente de tout ou partie des caractéristiques développées ci-dessus.

## Revendications

1. Procédé de préparation d'une unité de méthanisation, comportant dans l'ordre les étapes suivantes :
- la collecte de déchets verts,
- le compostage desdits déchets vert,
- le criblage des déchets verts issu du compostage, le criblage se faisant à une dimension comprise entre 30 et 80mm de sorte à éliminer les particules fines de déchets verts,
- l'insertion des déchets verts issus du criblage dans ladite unité de méthanisation, de sorte à former un garnissage structuré, qui est un support de culture (1) constitué à plus de 50 % d'éléments bois (2) dont au moins une dimension est supérieure à 80 mm, la porosité dudit support de culture (1) étant supérieure à 50%.

2. Procédé de préparation d'une unité de méthanisation selon la revendication précédente, comportant une étape de broyage après l'étape de collecte et avant l'étape de criblage, le broyage étant un broyage lent, de sorte à obtenir qu'une majorité d'éléments broyés ait une plus grande dimension inférieure au mètre.

3. Procédé de méthanisation d'effluents liquides à garnissage structuré, ledit procédé comprenant :
- la préparation d'une unité de méthanisation par un procédé selon l'une quelconque des revendications 1 ou 2, puis
- une étape de méthanisation d'effluents liquides dans ladite unité de méthanisation.

4. Procédé de méthanisation d'effluents liquides à garnissage structuré selon la revendication précédente, comprenant dans l'ordre les étapes suivantes :
- insertion d'un support de culture (1) dans un tunnel de méthanisation amont (3a),
- envoi d'effluents liquides dans le tunnel de méthanisation amont (3a),
- insertion d'un support de culture (1) dans un tunnel de méthanisation aval (3b),
- envoi des effluents traités en sortie du tunnel de méthanisation amont (3a) dans le tunnel de méthanisation aval (3b).

5. Procédé de méthanisation d'effluents liquides à garnissage structuré selon la revendication précédente, dans lequel une partie comprise entre 30% et 60% des effluents traités en sortie du tunnel de méthanisation aval (3b) est renvoyée en entrée du tunnel de méthanisation amont (3a).

6. Utilisation pour un procédé de méthanisation d'effluents liquides à garnissage structuré d'un support de culture (1) obtenu par le procédé suivant :
- la collecte de déchets verts,
- le compostage desdits déchets vert,
- le criblage des déchets verts issu du compostage, le criblage se faisant à une dimension comprise entre 30 et 80mm de sorte à éliminer les particules fines de déchets verts,
- formation dudit support de culture (1) à partir des déchets verts issus du criblage,
ledit support de culture (1) étant constitué à plus de 50% d'éléments bois (2), dont au moins une dimension est supérieure à 80 mm, la porosité dudit support de culture (1) étant supérieure à 50%, et
ledit procédé de méthanisation se faisant dans une unité de méthanisation et comprenant une étape de préparation de ladite unité de méthanisation dans laquelle le support de culture (1) est inséré dans ladite unité de méthanisation de sorte à former un garnissage structuré.

## Patentansprüche

1. Verfahren zum Vorbereiten einer Methanisierungsanlage, das die folgenden Schritte in der Reihenfolge enthält:
- Sammeln von Grünabfällen,
- Kompostieren der Grünabfälle,
- Sieben der Grünabfälle aus der Kompostierung, wobei das Sieben mit einer Abmessung zwischen 30 und 80 mm durchgeführt wird, um die Feinpartikel von den Grünabfällen zu entfernen,
- Einbringen der Grünabfälle aus der Siebung in die Methanisierungsanlage, um eine strukturierte Füllung zu bilden, die ein Kultursubstrat (1) ist, das zu mehr als 50 % aus Holzelementen (2) besteht, von denen mindestens eine Abmessung größer als 80 mm ist, wobei die Porosität des Kultursubstrats (1) größer als 50 % ist.

2. Verfahren zum Vorbereiten einer Methanisierungsanlage nach dem vorhergehenden Anspruch, das einen Zerkleinerungsschritt nach dem Sammelschritt und vor dem Siebschritt enthält, wobei das Zerkleinern ein langsames Zerkleinern ist, um zu erreichen, dass eine Mehrheit der zerkleinerten Elemente eine größte Abmessung von weniger als einem Meter hat.

3. Verfahren zum Methanisieren flüssiger Abwässer mit strukturierter Füllung, wobei das Verfahren Folgendes umfasst:
- Vorbereiten einer Methanisierungsanlage nach einem Verfahren nach einem der Ansprüche 1 oder 2, dann
- einen Schritt des Methanisierens flüssiger Abwässer in der Methanisierungsanlage.

4. Verfahren zum Methanisieren flüssiger Abwässer mit strukturierter Füllung nach dem vorhergehenden Anspruch, das die folgenden Schritte in der Reihenfolge umfasst:
- Einbringen eines Kultursubstrats (1) in einen vorgelagerten Methanisierungstunnel (3a),
- Einleiten flüssiger Abwässer in den vorgelagerten Methanisierungstunnel (3a),
- Einbringen eines Kultursubstrats (1) in einen nachgelagerten Methanisierungstunnel (3b),
- Einleiten der behandelten Abwässer am Ausgang des vorgelagerten Methanisierungstunnels (3a) in den nachgelagerten Methanisierungstunnel (3b).

5. Verfahren zum Methanisieren flüssiger Abwässer mit strukturierter Füllung nach dem vorhergehenden Anspruch, wobei ein Teil zwischen 30 % und 60 % der behandelten Abwässer am Ausgang des nachgelagerten Methanisierungstunnels (3b) zum Eingang des vorgelagerten Methanisierungstunnels (3a) zurückgeführt wird.

6. Verwendung, für ein Verfahren zum Methanisieren flüssiger Abwässer mit strukturierter Füllung, eines Kultursubstrats (1), das durch das folgende Verfahren erhalten wird:
- Sammeln von Grünabfällen,
- Kompostieren der Grünabfälle,
- Sieben der Grünabfälle aus der Kompostierung, wobei das Sieben mit einer Abmessung zwischen 30 und 80 mm durchgeführt wird, um die Feinpartikel von den Grünabfällen zu entfernen,
- Bilden des Kultursubstrats (1) aus den Grünabfällen aus der Siebung,
wobei das Kultursubstrat (1) zu mehr als 50 % aus Holzelementen (2) besteht, von denen mindestens eine Abmessung größer als 80 mm ist, wobei die Porosität des Kultursubstrats (1) größer als 50 % ist, und
wobei das Methanisierungsverfahren in einer Methanisierungsanlage durchgeführt wird und einen Schritt zum Vorbereiten der Methanisierungsanlage umfasst, wobei das Kultursubstrat (1) in die Methanisierungsanlage eingebracht wird, um eine strukturierte Füllung zu bilden.

## Claims

1. Method for preparing a methanisation unit, comprising in order the following steps:
- collecting green waste,
- composting said green waste,
- screening of green waste from composting step, the screening being carried out at a size between 30 and 80mm so as to eliminate fine particles of green waste,
- inserting green waste from screening step into said methanisation unit so as to form a structured packing, said structure packing being a culture substrate (1) made up of more than 50% of wood elements (2) of which at least one dimension is greater than 80 mm, the porosity of said culture substrate (1) being greater than 50%.

2. Method for preparing a methanisation unit according to the preceding claim, comprising a grinding step after the collecting step and before the screening step, the grinding being a slow grinding, so as to obtain that a majority of the ground elements have a largest dimension that is less than a metre.

3. Method for methanising liquid effluents with structured packing, said method comprising:
- the preparation of a methanisation unit by a method according to any one of Claims 1 or 2, then
- a methanising step of liquid effluents in said methanisation unit.

4. Method for methanising liquid effluents with structured packing according to the preceding claim, comprising in order the following steps:
- inserting a culture substrate (1) into an upstream methanisation tunnel (3a),
- sending liquid effluents into the upstream methanisation tunnel (3a),
- inserting a culture substrate (1) into a downstream methanisation tunnel (3b),
- sending effluents treated at the output of the upstream methanisation tunnel (3a) into the downstream methanisation tunnel (3b).

5. Method for methanising liquid effluents with structured packing according to the preceding claim, wherein a portion comprised between 30% and 60% of the effluents treated at the output of the downstream methanisation tunnel (3b) is sent back as input of the upstream methanisation tunnel (3a).

6. Use for a method for methanising liquid effluents with structured packing of a culture substrate (1) obtained by the following process:
- collecting green waste,
- composting said green waste,
- screening of green waste from composting step, the screening being carried out at a size between 30 and 80mm so as to eliminate fine particles of green waste,
- formation of said culture substrate (1) from the green waste resulting from screening step,
said culture substrate (1) being made up of more than 50% of wood elements (2) of which at least one dimension is greater than 80 mm, the porosity of said culture substrate (1) being greater than 50%, and
said method for methanising taking place in a methanisation unit and comprising a step of preparing said methanisation unit in which the culture substrate (1) is inserted into said methanisation unit so as to form a structured packing.
